# EUROPEAN PATENT APPLICATION

(11) **EP 4 692 364 A1**
(43) Date of publication of application: **11.02.2026**
(21) Application number: 24780789.4
(22) Date of filing: 29.03.2024
(51) Int. Cl.: C12P 19/30, C12P 1/00

(54) **METHOD FOR PRODUCING SUBSTANCE**

(30) Priority: 29.03.2023 JP 2023053097
(71) Applicant: Mitsubishi Chemical Corporation, Tokyo 100-8251 (JP)
(72) Inventor: SUZUKI, Kumpei, Tokyo 100-8251 (JP); SATO, Eiji, Tokyo 100-8251 (JP); AKIYAMA, Takanori, Tokyo 100-8251 (JP)
(74) Representative: Hoffmann Eitle
(86) International application number: PCT/JP2024/013013
(87) International publication number: WO 2024/204694

(57) **Abstract**

An object of the present invention is to provide a method for producing a substance, which makes it possible to produce a substance in a simple and efficient manner by an enzymatic reaction using ATP, in which an ATP regeneration reaction is coupled. The method for producing a substance according to an example of the embodiment is a method for producing a substance by an enzymatic reaction using ATP, in which the enzymatic reaction and an ATP regeneration reaction of reacting ADP or AMP with a phosphate donor to generate ATP are carried out in one reaction solution in the presence of a basic compound containing a divalent metal.

## Description

### TECHNICAL FIELD

The present invention relates to a method for producing a substance by carrying out an ATP regeneration reaction and an enzymatic reaction using ATP in one reaction solution, the method including carrying out the ATP regeneration reaction and the enzymatic reaction in the presence of a basic compound containing a divalent metal.

Priority is claimed on Japanese Patent Application No. 2023-053097, filed March 29, 2023, the content of which is incorporated herein by reference.

### BACKGROUND ART

Adenosine 5'-triphosphate (ATP) is used in a variety of enzymatic reactions as an energy source and plays a variety of roles in the synthesis of biological compounds. However, in a case where an enzymatic reaction that requires ATP is applied to industrial production of substances, ATP is very expensive, which is a significant economic burden. Therefore, a method of conjugating with an ATP regeneration system that re-synthesizes ATP from adenosine 5'-diphosphate (ADP) or adenosine 5'-monophosphate (AMP) generated by hydrolysis of ATP has been studied.

Creatine kinase in which creatine phosphate is a phosphate group donor, acetate kinase in which acetyl phosphate is a phosphate group donor, polyphosphate kinase in which polyphosphate is a phosphate group donor, or the like is known as an enzyme that catalyzes a reaction of regenerating ADP to ATP. On the other hand, the methods disclosed in Patent Documents 1 to 4 are known as methods for regenerating ATP from AMP. Above all, the ATP regeneration reaction using polyphosphate as a phosphate donor by the polyphosphate kinase type 2 family, which is disclosed in Patent Document 4, is known to be capable of regenerating ATP from AMP and ADP by a single enzyme.

Further, Patent Document 4 also discloses that, in a method for producing a substance by an enzymatic reaction using ATP, ATP regenerated from AMP and ADP is used for the synthesis of a target compound by conjugating an ATP regeneration reaction. In addition, Patent Documents 5 to 7 and Non-Patent Documents 1 to 3 disclose methods for synthesizing β-nicotinamide mononucleotide, oxidized glutathione, inosine monophosphate, and glycerol triphosphate by similar methods.

In addition, it is known that, in a case where ATP is used as an energy source in various enzymatic reactions, ATP binds to a divalent metal ion such as an Mg ion (Mg²⁺) to form a stable complex. Various divalent metal ions are also used in the production of a substance in which the ATP regeneration reaction is coupled as disclosed in Patent Documents 5 to 7 and Non-Patent Documents 1 to 3. Non-Patent Document 4 discloses a method for efficiently producing ATP by controlling an amount of Mg ions present in a reaction solution. In addition, Non-Patent Document 5 discloses that, in a case where a target substance is obtained by an enzymatic reaction using ATP, the enzyme activity is significantly improved by adding a very small excess (2 mM) of Mg ions relative to the equivalent amount of ATP. On the other hand, Non-Patent Documents 6 and 7 show that excess Mg ions inhibit an enzymatic reaction that uses ATP.

In general, in the production of a substance using an enzymatic reaction, conditions that maintain the pH are selected in order to prevent inactivation and denaturation of an enzyme or to keep the reaction within an optimum range. In order to maintain the pH, a component having a buffering capacity is allowed to coexist, or an acid or a base is added depending on the pH that changes with the reaction. Even in the production of a substance by an enzymatic reaction using ATP in which an ATP regeneration reaction is coupled, it is preferable to select conditions under which the pH is maintained.

For example, the pH is maintained using a HEPES-NaOH buffer solution at a molar concentration of 10 or more times than that of β-nicotinamide mononucleotide as a target substance, in Patent Document 5; a Tris-HCl buffer solution at an equimolar concentration or more than that of inosine monophosphate as a target substance, in Non-Patent Document 1; a MOPS-KOH buffer solution or a HEPES-KOH buffer solution at a molar concentration of 2 times that of glycerol triphosphate as a target substance, in Non-Patent Document 2; and a Tris-HCl buffer solution at a molar concentration of 5 or more times that of L-glutathione as a target substance, in Non-Patent Document 3.

In the production of a substance by an enzymatic reaction using ATP coupled with an ATP regeneration reaction, the pH changes due to the exchange of phosphate groups between substances that accompany the reaction and the generation of by-products. That is, in order to improve productivity, a correspondingly large amount of a buffer component is required, which poses problems in terms of purification load and cost.

In addition, as a method for maintaining the pH without using the buffer solution as described above, there is a method of adding an acid or a base depending on the pH that changes with the reaction. From the viewpoint of cost and ease of availability, HCl or H₂SO₄, or NaOH or KOH is frequently used as the acid or the base. However, in enzymatic reactions using ATP, there is no example in which the pH that changes with the reaction is maintained by intermittently or continuously adding such an inexpensive acid or base.

### Citation List

### Patent Documents

Patent Document 1: Japanese Unexamined Patent Application, First Publication No. 2001-299390
Patent Document 2: Japanese Unexamined Patent Application, First Publication No. 2005-46010
Patent Document 3: Japanese Unexamined Patent Application, First Publication No. 2006-81506
Patent Document 4: Japanese Unexamined Patent Application, First Publication No. 2013-21967
Patent Document 5: Japanese Unexamined Patent Application, First Publication No. 2022-25128
Patent Document 6: PCT International Publication No. WO2016/002884
Patent Document 7: PCT International Publication No. WO2018/203482
Patent Document 8: Japanese Unexamined Patent Application, First Publication No. 2020-22433

### Non Patent Documents

Non Patent Document 1: Scism, Robert A. and Bachmann, Brian O. "Five-component cascade synthesis of nucleotide analogues in an engineered self-immobilized enzyme aggregate." Chem. Bio. Chem. 11.1, 67-70, 2010
Non Patent Document 2: Restiawaty, Elvi et al. "Feasibility of thermophilic adenosine triphosphate-regeneration system using Thermus thermophilus polyphosphate kinase." Process Biochem. 46.9, 1747-1752, 2011
Non Patent Document 3: Zhang, Xing et al. "One-pot synthesis of glutathione by a two-enzyme cascade using a thermophilic ATP regeneration system." Journal of Biotechnology 241, 163-169, 2017
Non Patent Document 4: Tatsuro Fujio, "Construction of practical ATP regeneration system and its application to production of nucleotides", JSBBA Award for Achievement in Technological Research, Nippon Nogeikagaku Kaishi 66, 10, 1457-1465, 1992
Non Patent Document 5: K. Arnvig, B.H. Jensen and R. L. Switzer "Purification and properties of phosphoribosyl-diphosphate synthetase from Bacillus subtilis" Eur. J. Biochem. 192, 195-200, 1990
Non Patent Document 6: J. Park and R.S. Gupta "Adenosine kinase and Ribokinase - the RK family of proteins." Cell. Mol. Life Sci. 65, 2875-2896, 2008
Non Patent Document 7: Palella, T.D. et al. "Human Placental Adenosine Kinase. Kinetic mechanism and inhabitation." J. Biol. Chem. 255, 5264-5269. 1980

### SUMMARY OF INVENTION

### Technical Problem

As described above, even in the method for producing a substance by an enzymatic reaction using ATP coupled with an ATP regeneration reaction, it is preferable to take some measures for maintaining the pH in the same manner as in other enzymatic reactions. However, the method of maintaining the pH by the coexistence of a buffer solution has problems in terms of productivity, raw material costs, and purification load of a target substance.

A main object of the present invention is to provide a method for producing a substance, which makes it possible to produce a substance in a simple and efficient manner by an enzymatic reaction using ATP, in which an ATP regeneration reaction is coupled.

### Solution to Problem

In order to achieve the above-mentioned object, the present inventors first conducted studies on a method of maintaining the pH without relying on a large amount of a buffer component. That is, the present inventors examined to maintain the pH of an ATP regeneration reaction and an enzymatic reaction using ATP in the optimum range for an enzymatic reaction using NaOH, which is most frequently used as a pH-maintaining agent. However, the expected yield and accumulation concentration could not be obtained with NaOH.

As a result of further studies, the present inventors found that, in a method for producing a substance by an enzymatic reaction using ATP coupled with an ATP regeneration reaction, the ATP regeneration reaction and the enzymatic reaction can be carried out using a basic compound containing a divalent metal, and the pH during the reaction is maintained, thereby improving the reaction yield, accumulation concentration, and productivity of a target substance, thus completing the present invention.

That is, the present invention includes the following configurations.
[1] A method for producing a substance by an enzymatic reaction using adenosine 5'-triphosphate, the method comprising: carrying out the enzymatic reaction and an ATP regeneration reaction in which adenosine 5'-diphosphate or adenosine 5'-monophosphate is reacted with a phosphate donor to generate adenosine 5'-triphosphate, in one reaction solution in the presence of a basic compound containing a divalent metal.
[2] A method for producing a substance, comprising: producing β-nicotinamide mononucleotide using D-ribose, nicotinamide, and adenosine 5'-triphosphate in the presence of ribokinase, phosphoribosyl pyrophosphate synthetase, nicotinamide phosphoribosyltransferase, polyphosphate kinase, and a basic compound containing a divalent metal.
[3] A method for producing a substance by an enzymatic reaction using adenosine 5'-triphosphate, the method comprising: carrying out the enzymatic reaction and an ATP regeneration reaction in which adenosine 5'-diphosphate or adenosine 5'-monophosphate is reacted with a phosphate donor to generate adenosine 5'-triphosphate, in one reaction solution, wherein the pH of the reaction solution is adjusted with a basic compound containing a divalent metal.
[4] The method for producing a substance according to any one of [1] to [3], wherein the basic compound is at least one selected from basic compounds containing magnesium, calcium, strontium, barium, manganese, iron, cobalt, nickel, or zinc as the divalent metal.
[5] The method for producing a substance according to any one of [1] to [4], wherein the basic compound containing a divalent metal is at least one selected from magnesium hydroxide and magnesium oxide.
[6] The method for producing a substance according to any one of [1] or [3] to [5], wherein the ATP regeneration reaction is an ATP regeneration reaction by polyphosphate kinase (Ppk).
[7] The method for producing a substance according to any one of [1] or [3] to [6], wherein the enzymatic reaction is an enzymatic reaction using at least one enzyme selected from enzymes belonging to EC number EC 2.4.-, EC 2.5.-, EC 2.7.-, EC 6.2.-, or EC 6.3.-.
[8] The method for producing a substance according to [6], wherein the enzymatic reaction is an enzymatic reaction using at least one enzyme selected from L-amino-acid alpha-ligase, ribokinase, phosphoribosyl pyrophosphate synthetase, and nicotinamide phosphoribosyltransferase.
[9] The method for producing a substance according to [6], wherein the enzymatic reaction is an enzymatic reaction in which ribokinase, phosphoribosyl pyrophosphate synthetase, and nicotinamide phosphoribosyltransferase are combined.
[10] The method for producing a substance according to [6], wherein the enzymatic reaction is an enzymatic reaction in which xylose isomerase, xylulokinase, ribulose-5-phosphate-3-epimerase, ribose-5-phosphate isomerase, phosphoribosyl pyrophosphate synthetase, and nicotinamide phosphoribosyltransferase are combined.
[11] The method for producing a substance according to any one of [1] or [3] to [10], wherein the total concentration of adenosine 5'-triphosphate, adenosine 5'-diphosphate, and adenosine 5'-monophosphate in the reaction solution is 0.1 mM to 3 mM.
[12] The method for producing a substance according to any one of [1] or [3] to [11], wherein the pH of the reaction solution is maintained at 6.0 to 9.0 using the basic compound containing a divalent metal.
[13] The method for producing a substance according to any one of [1] or [3] to [12], wherein the substance produced by the enzymatic reaction is at least one selected from a nucleotide, an amino acid, a peptide, and a phosphate compound.
[14] The method for producing a substance according to any one of [1] or [3] to [12], wherein the substance produced by the enzymatic reaction is β-nicotinamide mononucleotide, inosine monophosphate, carnosine, aspartic acid, glutathione, oxidized glutathione, γ-glutamylcysteine, phosphoribosyl pyrophosphate, ribose-5-phosphate, glycerol triphosphate, acetyl CoA, or S-adenosylmethionine.

### Advantageous Effects of Invention

According to the present invention, it is possible to simply and efficiently produce a substance by an enzymatic reaction using ATP, in which an ATP regeneration reaction is coupled.

### BRIEF DESCRIPTION OF DRAWINGS

[FIG. 1A] A graph showing an amount of a pH-maintaining agent added over time (broken line) and a transition in pH (solid line) in Example 1.
[FIG. 1B] A graph showing an amount of a pH-maintaining agent added over time (broken line) and a transition in pH (solid line) in Example 2.
[FIG. 1C] A graph showing an amount of a pH-maintaining agent added over time (broken line) and a transition in pH (solid line) in Comparative Example 1.
[FIG. 2A] A graph showing an amount of a pH-maintaining agent added over time (broken line) and a transition in pH (solid line) in Example 3.
[FIG. 2B] A graph showing an amount of a pH-maintaining agent added over time (broken line) and a transition in pH (solid line) in Comparative Example 2.
[FIG. 3] A graph showing a transition in NMN synthesis yield in a case where the pH is maintained by a pH-maintaining agent in Examples 1 and 2 and Comparative Example 1.
[FIG. 4] A graph showing a transition in L-carnosine synthesis yield in a case where the pH is maintained by a pH-maintaining agent in Example 3 and Comparative Example 2.
[FIG. 5A] A graph showing an amount of a pH-maintaining agent added over time (broken line) and a transition in pH (solid line) in Example 4.
[FIG. 5B] A graph showing an amount of a pH-maintaining agent added over time (broken line) and a transition in pH (solid line) in Example 5.
[FIG. 6] A graph showing a transition in NMN synthesis yield in a case where the pH is maintained by a pH-maintaining agent in Examples 4 and 5.

### DESCRIPTION OF EMBODIMENTS

Hereinafter, the present invention will be described in more detail. The following embodiments are merely examples for explaining the present invention, and are not intended to limit the present invention thereto. The present invention can be carried out in various forms without departing from the spirit and scope of the present invention.

The following definitions of terms are adopted in the present specification.

Any numerical range expressed by "to" means a numerical range including numerical values before and after "to" as a lower limit value and an upper limit value, respectively.

The numerical ranges of the contents, various physical property values, and property values disclosed in the present specification can be any new numerical ranges of any combinations of the lower limit values and the upper limit values thereof.

The definitions of the abbreviations used in the present specification are as follows.
Nampt: nicotinamide phosphoribosyltransferase
Prs: phosphoribosyl pyrophosphate synthetase
Rbk: ribokinase
Ppk: polyphosphate kinase
PPase: pyrophosphatase
Lal: L-amino-acid alpha-ligase
NMN: β-nicotinamide mononucleotide
PRPP: phosphoribosyl pyrophosphate
NAM: nicotinamide
R5P: ribose-5-phosphate
NR: nicotinamide riboside
NaMN: nicotinic acid mononucleotide
NAD: nicotinamide adenine dinucleotide
PPi: pyrophosphate
PolyP: polyphosphate
ATP: adenosine 5'-triphosphate
ADP: adenosine 5'-diphosphate
AMP: adenosine 5'-monophosphate
Pi: phosphoric acid
IMP: inosine monophospate
GMP: guanosine monophospate
XMP: xanthosine monophosphate
SAM: S-adenosylmethionine

Hereinafter, each of the configurations of the present invention will be described in detail.

The method for producing a substance according to the embodiment is a method for producing a substance by an enzymatic reaction using ATP, in which the enzymatic reaction and an ATP regeneration reaction of reacting ADP or AMP with a phosphate donor to generate ATP are carried out in one reaction solution in the presence of a basic compound containing a divalent metal. That is, the method for producing a substance according to the embodiment is a method for producing a substance by an enzymatic reaction using ATP, in which the enzymatic reaction and the ATP regeneration reaction are coupled in the presence of a basic compound containing a divalent metal to produce a target substance.

In the present invention, the term "basic compound containing a divalent metal" refers to a metal salt that generates a divalent metal ion in a case of being dissolved in water and exhibits basicity (a pH of greater than 7.0). The pH of the reaction solution can be adjusted using the basic compound containing a divalent metal.

In a suitable example, in a case where the ATP regeneration reaction and the enzymatic reaction using ATP are carried out in one reaction solution, the pH of the reaction solution is adjusted by the basic compound containing a divalent metal. More preferably, the basic compound containing a divalent metal is sequentially added during the reaction to maintain the pH of the reaction solution within an optimum range.

The method for producing a substance according to the embodiment can be used for the production of at least one selected from, for example, a nucleotide, an amino acid, a peptide, and a phosphate compound and is particularly useful for the production of NMN.

It is possible to simply and efficiently produce a substance by carrying out an ATP regeneration reaction and an enzymatic reaction using ATP in one reaction solution in the presence of a basic compound containing a divalent metal.

The specific mechanism by which the yield of the substance is improved by carrying out the ATP regeneration reaction and the enzymatic reaction using ATP while maintaining the pH by adding the basic compound containing a divalent metal is not clear, but it is presumed that one or more of the following (i) to (iv) are involved.
(i) The addition of a basic compound containing a divalent metal maintains the amount of divalent metal ions (particularly Mg ions) present at an appropriate level, thereby promoting the formation of a complex of ATP and a divalent metal.
(ii) The interaction between divalent metal ions and phosphate compounds such as phosphoric acid and pyrophosphate, which may be generated as by-products, alleviates the inhibition of enzymatic reactions by the phosphate compounds.
(iii) The divalent metal ions interact with not only ATP but also a product or substrate compound having a phosphate group, thereby stabilizing the product or substrate compound or making the product or substrate compound more available to an enzyme.
(iv) In the presence of an appropriate amount of divalent metal ions, the enzyme itself that utilizes ATP easily takes an active form.

### <Basic compound containing divalent metal>

Examples of the divalent metal contained in the basic compound include magnesium, calcium, strontium, barium, manganese, iron, cobalt, nickel, and zinc. That is, examples of the basic compound containing a divalent metal include a basic magnesium compound, a basic calcium compound, a basic strontium compound, a basic barium compound, a basic manganese compound, a basic iron compound, a basic cobalt compound, a basic nickel compound, and a basic zinc compound. The basic compound containing a divalent metal may be used alone or in combination of two or more thereof.

The basic magnesium compound is not particularly limited and examples thereof include magnesium hydroxide, magnesium oxide, magnesium peroxide, magnesium carbonate, and mixtures thereof.

The basic calcium compound is not particularly limited and examples thereof include calcium hydroxide and calcium oxide.

The basic strontium compound is not particularly limited and examples thereof include strontium hydroxide and strontium oxide.

The basic barium compound is not particularly limited and examples thereof include barium hydroxide and barium oxide.

The basic manganese compound is not particularly limited and examples thereof include divalent manganese hydroxide, manganese oxide, and manganese carbonate.

The basic iron compound is not particularly limited and examples thereof include iron (II) hydroxide.

The basic cobalt compound is not particularly limited and examples thereof include cobalt (II) hydroxide.

The basic nickel compound is not particularly limited and examples thereof include nickel (II) hydroxide.

The basic zinc compound is not particularly limited and examples thereof include zinc hydroxide.

From the viewpoint of a high effect of improving the yield of the substance, the basic compound containing a divalent metal is preferably at least one obtained from basic compounds containing magnesium, calcium, strontium, barium, manganese, iron, cobalt, nickel, or zinc as the divalent metal, more preferably a basic magnesium compound or a basic manganese compound, and particularly preferably a basic magnesium compound. Above all, at least one selected from magnesium hydroxide and magnesium oxide is most preferable.

The basic compound containing a divalent metal may be added in the form of a powder, may be added in the form of an aqueous solution, or may be added in the form of a dispersed slurry.

The method of adding the basic compound containing a divalent metal is preferably a method in which the pH of the reaction solution can be maintained in an optimum range, but the method is not limited thereto. The basic compound containing a divalent metal may be added intermittently or continuously.

In a suitable example, it is preferable to adjust the pH of the reaction solution to a range in which the catalytic efficiency of the ATP regeneration reaction and the enzymatic reaction using ATP is optimal by adding the basic compound containing a divalent metal during the reaction.

Since many enzymatic reactions have an optimum pH in a neutral range, the pH maintenance range of the reaction solution by the basic compound containing a divalent metal is preferably 6.0 to 9.0 and more preferably 6.5 to 8.5.

The time for which the pH is maintained in the optimum range by adding the basic compound containing a divalent metal can be set to a time until the amount of the target substance generated reaches a predetermined amount. The time for which the pH is maintained from the start of the reaction can be set to, for example, 1 to 48 hours and is preferably 1 to 24 hours.

The amount of the basic compound containing a divalent metal to be added may be any amount as long as the pH of the reaction solution can be maintained in a range in which the catalytic efficiency of the ATP regeneration reaction and the coupled enzymatic reaction using ATP is optimal. The amount of the basic compound containing a divalent metal to be added can be set to, for example, 0.1 to 10 equivalents and is preferably 1 to 10 equivalents with respect to the phosphate group or pyrophosphate group transferred by the enzymatic reaction and the by-produced phosphate compound.

### <ATP regeneration reaction>

The ATP regeneration reaction in the production method according to the embodiment is not particularly limited as long as it is a reaction which is capable of obtaining ATP from ADP or AMP, and may be either a method to which a biological reaction is applied or a method to which chemical synthesis is applied. Since the regenerated ATP is used in an enzymatic reaction, a method in which a biological reaction that has mild conditions common to the enzymatic reaction is applied is preferable. Specifically, examples of the enzyme that catalyzes the reaction of regenerating ADP into ATP include creatine kinase that uses creatine phosphate as a phosphate group donor, acetate kinase that uses acetyl phosphate as a phosphate group donor, and polyphosphate kinase (Ppk) that uses polyphosphate as a phosphate group donor. Above all, an ATP regeneration reaction using Ppk is preferable since polyphosphate used as a phosphate donor, which is a substrate, is inexpensive.

Ppk can be classified into two families of polyphosphate kinase type 1 family (Ppk1) and polyphosphate kinase type 2 family (Ppk2), based on differences in amino acid sequence and kinetics. The activity of regenerating ATP using polyphosphate as a substrate is higher in Ppk2 than in Ppk1. Therefore, it is preferable to use Ppk2 as Ppk.

Ppk2 can be further classified into three subfamilies, that is, class 1, class 2, and class 3. Ppk2 class 1 and Ppk2 class 2 each catalyze a reaction of phosphorylating ADP to generate ATP and a reaction of phosphorylating AMP to generate ADP. On the other hand, Ppk2 class 3 can catalyze both a phosphorylation reaction of AMP and a phosphorylation reaction of ADP, and can therefore solely generate ATP from AMP.

In a case where a by-product generated in the enzymatic reaction using ATP is ADP, it is preferable to select Ppk2 class 1 which is capable of regenerating ATP from ADP or Ppk2 class 3 which is capable of regenerating ATP from both ADP and AMP as Ppk. In a case where a by-product generated in the enzymatic reaction using ATP is AMP, it is preferable to select a combination of Ppk2 class 2, which is capable of regenerating ATP from AMP, and Ppk2 class 1, or Ppk2 class 3.

In a method for producing a substance in one pot by a plurality of enzymatic reactions using ATP, a combination of Ppk2 class 1 for regenerating ATP from ADP and Ppk2 class 2 for regenerating ADP from AMP can be used in a reaction system in which both ADP and AMP are by-produced from ATP. However, it is preferable to use Ppk2 class 3 from the viewpoint of high efficiency of being able to solely catalyze both the reaction of regeneration of ATP from ADP and the reaction of regeneration of ATP from AMP. In addition, the coexistence of adenylate kinase is also effective.

The origin of Ppk is not particularly limited and examples of Ppk2 class 1 include those derived from Rhodobacter sphaeroides, Sinorhizobium meliloti, Pseudomonas aeruginosa, and Francisella tularensis.

Examples of Ppk2 class 2 include those derived from Pseudomonas aeruginosa.

Examples of Ppk2 class 3 include those derived from Deinococcus radiodurans, Paenarthrobacter aurescens, Meiothermus rube, Deinococcus geothermalis, and Thermosynechococcus elongatus.

With regard to Ppk, not only wild type Ppk, but also improved mutants or recombinants in which, for example, gene transcription and translation activities or enzyme activities have been altered can be used.

The form of Ppk used in the reaction may be any form as long as it has its activity, and Ppk may be used in the form of living cells of a living organism as they are, dead cells, or proteins that has been separated from cells and purified. In a case where purified proteins are used, the degree of purification of the proteins having Ppk activity is not particularly limited, and the proteins may be crudely purified, may be a disrupted suspension of bacterial cells that has been simply disrupted by ultrasonication or a high-pressure homogenizer, or may be purified proteins purified by affinity chromatography or the like. In addition, the proteins may be in the form of freeze-dried bacterial cells, acetone-dried bacterial cells, or a ground product thereof, of cells having a Ppk activity, or may be in the immobilized form of proteins themselves or bacterial cells.

The concentration of Ppk in the reaction solution can be adjusted, and it is preferable to appropriately adjust the concentration of Ppk depending on the amount of the coupled enzymatic reaction using ATP. The concentration of Ppk in the reaction solution can be set to, for example, 1 to 10 g/L and is preferably 0.1 to 10 g/L.

"Polyphosphate", which is a phosphate donor of Ppk, means a polymer of high-molecular-weight phosphate formed by dehydration condensation of phosphoric acid and a salt thereof.

The polyphosphate is not particularly limited as long as it acts as a substrate for Ppk, and examples thereof include polyphosphate, metaphosphate, ultraphosphate, cyclopolyphosphate, salts thereof, and mixtures thereof. It may also be referred to as a Graham's salt, a Maddrell's salt, a Kurrol's salt, or the like. As the salt of polyphosphate, a sodium salt is preferable from the viewpoint of cost and solubility.

The degree of polymerization of polyphosphate is not particularly limited, and a mixture of polyphosphates having different degrees of polymerization may be used. A mixture of polyphosphates having a single chain-like structure, a cyclic structure, and a three-dimensional network structure may also be used. Patent Document 7 discloses that a target reaction proceeds at a high conversion rate using a mixture containing polyphosphates having a high degree of polymerization as a phosphate donor for Ppk2, and a polyphosphate having a high degree of polymerization is preferable for carrying out an efficient ATP regeneration reaction.

The polyphosphate may be added in the form of a powder, may be added in the form of an aqueous solution, or may be added in the form of a slurry after being dispersed.

The concentration of the polyphosphate in the reaction solution is preferably adjusted depending on the activity of the ATP regeneration reaction and the activity of the enzymatic reaction using ATP, and can be set to, for example, 0.1 to 250 g/L and is preferably 0.1 to 25 g/L. The required amount of the polyphosphate may be added to the reaction solution at the start of the reaction, but the polyphosphate may be intermittently or continuously added to maintain the concentration in a range of 0.1 to 250 g/L since the polyphosphate is consumed in the ATP regeneration reaction. Patent Document 5 discloses that a lower concentration of polyphosphate results in a higher generation concentration of the target substance, and it is more preferable to add the polyphosphate intermittently or continuously to maintain the concentration in a range of 0.1 to 25 g/L.

### <Enzymatic reaction using ATP>

The enzymatic reaction using ATP, which is coupled with the ATP regeneration reaction, is not particularly limited as long as it is a reaction that consumes ATP to by-produce ADP or AMP, and refers to an enzymatic reaction that is catalyzed using the free energy generated by the hydrolysis of ATP or an enzymatic reaction that transfers a phosphate group or a pyrophosphate group generated by the hydrolysis of ATP.

Examples of the enzyme used in the enzymatic reaction using ATP include a transferase that transfers a glycosyl group (EC 2.4.-), a transferase that transfers an alkyl group of an allyl group (EC 2.5.-), a transferase that transfers a group containing phosphorus (EC 2.7.-), a synthetase that forms a C-S bond (EC 6.2.-), and a synthetase that forms a C-N bond (EC 6.3.-).

The enzymatic reaction using ATP is preferably a reaction using at least one enzyme selected from the enzymes belonging to the EC number EC 2.4.-, EC 2.5.-, EC 2.7.-, EC 6.2.-, or EC 6.3.-.

Examples of the enzymatic reaction using ATP by a transferase that transfers a glycosyl group include the synthesis of IMP GMP, or XMP by hypoxanthine phosphoribosyltransferase (E.C. 2.4.2.8) and the synthesis of NMN by nicotinamide phosphoribosyltransferase (E.C. 2.4.2.12).

Examples of the enzymatic reaction using ATP by a transferase that transfers an alkyl group or an allyl group include the synthesis of SAM by S-adenosylmethionine synthetase (E.C. 2.5.1.6).

Examples of the enzymatic reaction using ATP by a transferase that transfers a group containing phosphorus include the synthesis of R5P by Rbk (E.C. 2.7.1.15), the synthesis of PRPP by Prs (E.C. 2.7.6.1), and the synthesis of glycerol triphosphate by glycerol kinase (E.C. 2.7.1.30).

Examples of the enzymatic reaction using ATP by a synthetase that forms a C-S bond include the synthesis of acetyl-CoA by acetyl-CoA synthetase (E.C. 6.2.1.1).

Examples of the enzymatic reaction using ATP by a synthetase that forms a C-N bond include the synthesis of a dipeptide by Lal (E.C. 6.3.2.28), the synthesis of glutathione or oxidized glutathione by glutathione synthetase (E.C. 6.3.2.3), the synthesis of γ-glutamylcysteine by glutamate-cysteine ligase (E.C. 6.3.2.2), and the synthesis of aspartic acid by asparagine synthetase (EC. 6.3.1.1). Examples of the synthesis of a dipeptide by Lal include the synthesis of carnosine by carnosine synthetase (E.C. 6.3.2.11).

The enzymatic reaction using ATP is preferably an enzymatic reaction using at least one enzyme selected from Lal, Rbk, Prs, and Nampt and more preferably an enzymatic reaction using a combination of Rbk, Prs, and Nampt. NMN can be produced from ribose through R5P and PRPP by an enzymatic reaction using a combination of Rbk, Prs, and Nampt.

Another example of the enzymatic reaction using ATP is an enzymatic reaction using a combination of xylose isomerase, xylulokinase, ribulose-5-phosphate-3-epimerase, ribose-5-phosphate isomerase, Prs, and Nampt. By an enzymatic reaction in which these enzymes are used in combination, NMN can be produced from xylose via R5P and PRPP.

Those skilled in the art can easily understand reactions other than the above-mentioned reactions by searching for, for example, KEGG (https://www.genome.jp/kegg/kegg_ja.html) regarding an enzymatic reaction that generates a substance using ATP. In addition, there is no particular limitation on the origin of these enzymes, and not only wild type enzymes but also improved mutants or recombinants in which, for example, gene transcription and translation activities or enzyme activities have been altered can be used.

Similar to Ppk, the form of the enzyme that uses ATP is not limited as long as it is in a form that can exhibit the desired activity, and the enzyme may be used in the form of living cells of a living organism as they are, dead cells, or proteins that has been separated from cells and purified. In a case where purified proteins are used, the degree of purification is not particularly limited, and the proteins may be crudely purified, may be a disrupted suspension of bacterial cell that has been simply disrupted by ultrasonication or a high-pressure homogenizer, or may be purified proteins purified by affinity chromatography or the like. In addition, the proteins may be in the form of freeze-dried bacterial cells, acetone-dried bacterial cells, or a ground product thereof, of cells having a desired activity, or may be in the immobilized form of proteins themselves or bacterial cells.

One of suitable examples of the method for producing a substance according to the embodiment is a method for producing NMN using D-ribose, NAM, and ATP in the presence of Rbk, Prs, Nampt, PpK, and a basic compound containing a divalent metal.

Since ATP is an expensive raw material, it is preferable that the amount of ATP added to the reaction solution be small. The concentration of ATP in the reaction solution is preferably adjusted depending on the activity of the enzymatic reaction using ATP and the activity of the ATP regeneration reaction, and can be set to, for example, 0.1 to 10 mM and is preferably 0.1 to 3 mM.

Since ATP is regenerated during the reaction by the coupled ATP regeneration reaction, it is not always necessary to add ATP at the start of the reaction, and ADP or AMP can be used instead. In the present invention, the reaction proceeds even in a case of using a catalytic amount of ATP, ADP, or AMP with respect to a substrate of the enzymatic reaction using ATP.

The total concentration of ATP, ADP, and AMP in the reaction solution can be set to, for example, 0.1 to 10 mM and is preferably 0.1 to 3 mM.

The total concentration of ATP, ADP, and AMP in the reaction solution can be set to 1/1000 to 1/10 equivalent and is preferably 1/1000 to 1/50 equivalent with respect to the substrate of the enzymatic reaction using ATP.

ATP, ADP, and AMP may be in the form of salts or free compounds. Furthermore, ATP, ADP, and AMP may be in the form of hydrates. In the enzymatic reaction using ATP, the amounts of compounds serving as substrates other than ATP and other raw material compounds can be adjusted, and it is preferable to adjust the amounts depending on the activity of the enzymatic reaction, the activity of the ATP regeneration reaction, and the concentration of ATP.

The concentration of the enzyme that uses ATP in the reaction solution can be appropriately adjusted. In a case where the enzymatic reaction using ATP is excessive with respect to the ATP regeneration reaction, the reaction stagnates due to a lack of ATP, and thus it is preferable to adjust the concentration of the enzyme using ATP depending on the concentrations of Ppk and ATP. The concentration of the enzyme that uses ATP can be set to, for example, 1 µg/L to 10 g/L and is preferably 0.1 g/L to 10 g/L.

In the enzymatic reaction using ATP, ATP is used in a state of binding to a divalent metal ion to form a stable complex. From this reason, in the production of a substance by an enzymatic reaction using ATP, conditions for the coexistence of divalent metal ions have been selected in many related art, and for example, magnesium chloride, magnesium sulfate, manganese chloride, or manganese sulfate is frequently used.

In the present embodiment, it is also preferable that divalent metal ions be allowed to coexist in the reaction solution at the start of the reaction, and the divalent metal salt not be particularly limited. For example, it is preferable to use magnesium sulfate, magnesium chloride, manganese sulfate, manganese chloride, or a basic magnesium compound. These divalent metal salts may be used alone or in combination of two or more thereof. In addition, these divalent metal salts may also be used in the form of a hydrate.

In a case where a basic compound containing a divalent metal is used in order to allow divalent metal ions to coexist at the start of the reaction, the basic compound may be the same as or different from the basic compound containing a divalent metal used as a pH-maintaining agent for adjusting the pH of the reaction solution during the reaction.

In the present embodiment, the amount of divalent metal salt used for the coexistence of divalent metal ions at the start of the reaction in the reaction solution can be appropriately adjusted, and it is preferable to add a divalent metal salt in an amount equimolar to or greater than that of ATP since the divalent metal salt forms a complex with ATP.

Hereinafter, as an example of an enzymatic reaction using ATP, which can be used as one embodiment of the present invention, reactions using Rbk (E.C. 2.7.1.15), Prs (E.C. 2.4.2.17), Nampt (E.C. 2.4.2.12), and Lal (E.C. 6.3.2.28) will be described.

### [Enzymatic reaction using Rbk]

As in the reaction represented by Formula (1), Rbk is known as a transferase that transfers phosphoric acid from ATP to ribose, generating R5P and ADP. Since the ADP generated by the reaction is regenerated to ATP by the coupled ATP regeneration reaction, R5P can be efficiently obtained by adding a catalytic amount of ATP, ADP, or AMP to the reaction solution.

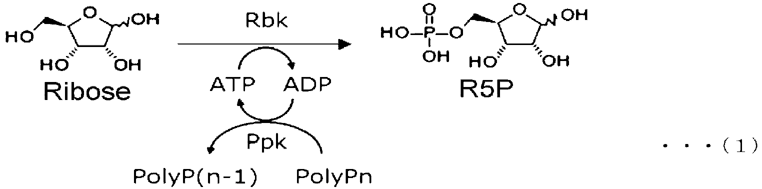

The origin of Rbk is not particularly limited as long as it exhibits an activity of catalyzing the reaction of Formula (1), and a naturally occurring Rbk derived from various organisms or a mutant Rbk constructed by modifying an amino acid sequence thereof can be used. Examples of the Rbk include Rbk derived from human (Homo sapiens), Rbk derived from yeast (Saccharomyces cerevisiae), Rbk derived from Bacillus subtilis, Rbk derived from Escherichia coli, Rbk derived from Haemophilus influenzae, and mutants thereof.

### [Enzymatic reaction using Prs]

As in the reaction represented by Formula (2), Prs is known as a transferase that transfers PPi from ATP to R5P, generating PRPP and AMP. Since the AMP generated by the reaction is regenerated to ATP by the coupled ATP regeneration reaction, PRPP can be efficiently obtained by adding a catalytic amount of ATP, ADP, or AMP to the reaction solution.

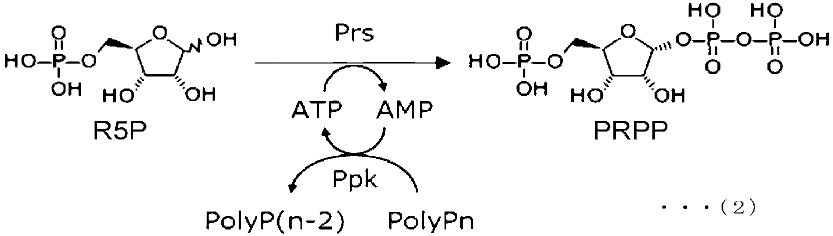

The origin of Prs is not particularly limited as long as it exhibits an activity of catalyzing the reaction of Formula (2), and a naturally occurring Prs derived from various organisms or a mutant Prs constructed by modifying an amino acid sequence thereof can be used. Examples of the Prs include Prs derived from human (Homo sapiens), Prs derived from Bacillus subtilis, Prs derived from Bacillus caldolyticus, Prs derived from Arabidopsis thaliana, Prs derived from Methanocaldococcus jannaschii, and mutants thereof. A mutant Prs as described in PCT International Publication No. WO2016/198948 can also be used.

### [Enzymatic reaction using Nampt]

Nampt is known as a transferase that catalyzes an exchange reaction of PPi of PRPP and NAM, generating NMN and PPi.

In principle, ATP is not essential for Nampt to catalyze the reaction. However, it has been reported that Nampt has an ATP hydrolysis activity, and enzymatic parameters or chemical equilibrium changes in a direction favorable for the generation of NMN by autophosphorylation of Nampt by the hydrolysis of ATP (E. S. Burgos and V. L. Schramm "Weak Coupling of ATP Hydrolysis to the Chemical Equilibrium of Human Nicotinamide Phosphoribosyltransferase" J. Biochem. 47, 11086-11096, 2008). Therefore, in the presence of ATP, Nampt generates NMN, PPi, and ADP from PRPP, NAM, and ATP as in the reaction represented by Formula (3). Since the ADP generated by the reaction is regenerated to ATP by the coupled ATP regeneration reaction, NMN can be efficiently obtained by adding a catalytic amount of ATP, ADP, or AMP to the reaction solvent.

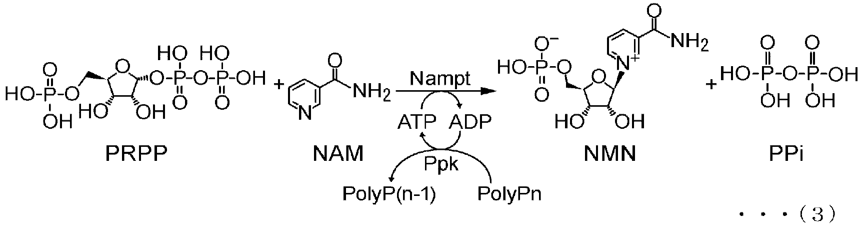

The origin of Nampt is not limited as long as it exhibits the above-mentioned activity, and a naturally occurring Nampt derived from various organisms or a mutant Nampt constructed by modifying an amino acid sequence thereof can be used. Examples of the Nampt include Nampt derived from human (Homo sapiens); Nampt derived from mouse (Mus musculus); Nampt derived from rat (Rattus norvegicus); Nampt derived from zebrafish (Danio rerio); Nampt derived from bacteria such as Nampt derived from the genus Haemophilus, suitably Haemophilus ducreyi; Nampt derived from the genus Deinococcus, suitably Deinococcus radiodurans; Nampt derived from the genus Oenococcus, suitably Oenococcus oeni; Nampt derived from the genus Shewanella, suitably Shewanella oneidensis; Nampt derived from the genus Vibrio, suitably Vibrio bacteriophage; Nampt derived from the genus Xanthomonas, suitably Xanthomonas translucens; Nampt derived from the genus Aquimonas, suitably Aquimonas voraii; Nampt derived from the genus Stenotrophomonas; Nampt derived from the genus Meiothermus, suitably Meiothermus ruber; Nampt derived from the genus Sphingopyxis; Nampt derived from the genus Chitinophaga, suitably Chitinophaga pinensis; and Nampt derived from the genus Caulobacter, suitably Caulobacter flavus; and mutants thereof.

In addition, Patent Document 5 discloses that the reaction of Formula (3) can be significantly promoted by decomposing the by-produced PPi. The PPi-decomposing reaction by PPase, which is an enzyme that decomposes PPi, is, for example, a reaction represented by Formula (4).

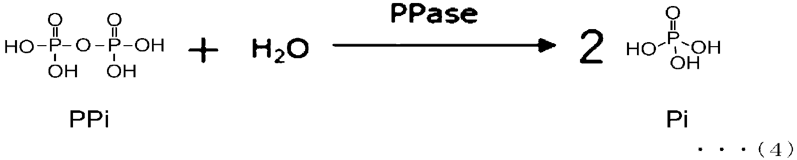

In the production method according to the embodiment, it is preferable that the reaction of Formula (4) be coupled with the reaction of Formula (3).

The origin of PPase is not limited as long as it exhibits the above-mentioned activity, and a naturally occurring PPase derived from various organisms or a mutant PPase constructed by modifying an amino acid sequence thereof can be used. Examples of PPase include PPase derived from yeast (Saccharomyces cerevisiae), PPase derived from Escherichia coli, PPase derived from Bacillus subtilis, PPase derived from Thermus thermophilus, PPase derived from Streptococcus gordonii, PPase derived from Streptococcus mutans, and mutants thereof.

PPase may be in any form as long as it can be coupled to the reaction of Formula (3) and may be prepared by any method. A commercially available purified PPase enzyme can also be used. Examples of the commercially available purified PPase enzyme include a yeast-derived purified PPase enzyme (product number: 10108987001) manufactured by Sigma-Aldrich Co. LLC.

In a case where PPase is used, the amount of PPase to be coexisted in the reaction solution may be such that sufficient PPase activity is present with respect to the Nampt activity, and can be set to, for example, 1 µg/L to 10 g/L.

Patent Document 5 discloses a method for producing NMN in a cascade manner in a single reactor by combining the reactions of Formulae (1) to (4). In one embodiment of the present invention, the target compound may be produced by carrying out the reactions of Formulae (1), (2), and (3) alone in the presence of a basic compound containing a divalent metal, or the target compound may be produced by carrying out the reactions of Formulae (1), (2), and (3) in combination in the presence of a basic compound containing a divalent metal in a single reactor. Suitably, it is preferable to synthesize NMN by combining the reactions of Formulae (1) to (4) in the presence of a basic compound containing a divalent metal.

### [Enzymatic reaction using Lal]

Lal is known as a synthetase that generates a peptide bond by directly linking two unprotected amino acid molecules to each other in conjugation with the hydrolysis reaction of ATP. Regarding the substrate specificity thereof, it is known that it is possible to synthesize a dipeptide or an oligopeptide and derivatives thereof from a wide variety of combinations of amino acids and condensates thereof.

By applying the enzymatic reaction using Lal, it is possible to generate a target dipeptide or oligopeptide and ADP from ATP and a combination selected from amino acids and condensates thereof. Since the ADP generated by the reaction is regenerated to ATP by the coupled ATP regeneration reaction, a dipeptide or an oligopeptide can be efficiently obtained by adding a catalytic amount of ATP, ADP, or AMP to the reaction solution.

The origin of Lal is not particularly limited, and a naturally occurring Lal derived from various organisms or a mutant Lal constructed by modifying an amino acid sequence thereof can be used. Examples of Lal include Lal derived from Bacillus subtilis, Lal derived from Pseudomonas syringae, Lal derived from Streptomyces tendae, Lal derived from Ralstonia solanacearum, Lal derived from Actinobacillus pleuropneumoniae, Lal derived from Chromobacterium violaceum, Lal derived from Photorhabdus luminescens, Lal derived from Bacillus licheniformis, and mutants thereof.

Japanese Unexamined Patent Application, First Publication No. 2020-22433 discloses a method for efficiently producing a dipeptide or a dipeptide derivative from one or more types of amino acids or amino acid derivatives, the method being a production method using Lal, which is coupled with an ATP regeneration reaction using Ppk. In one embodiment of the present invention, as the method for synthesizing a dipeptide and a dipeptide derivative, it is preferable to carry out an ATP regeneration reaction using Ppk and an enzymatic reaction using Lal in one reaction solution in the presence of a basic compound containing a divalent metal.

The conditions for proceeding with the ATP regeneration reaction and the enzymatic reaction using ATP, for example, temperature and time can be appropriately adjusted.

The reaction temperature is preferably adjusted within a range in which the catalytic efficiency of the ATP regeneration reaction and the enzymatic reaction using ATP is optimal. Since the optimum temperature for many enzymatic reactions is around 30°C to 40°C, the reaction temperature is preferably 20°C to 50°C and more preferably 30°C to 40°C.

The reaction time may be such that the amount of the target substance generated reaches a predetermined amount, and can be set to, for example, 1 to 48 hours and is preferably 1 to 24 hours.

### [Examples]

The present invention will be specifically described below with reference to Examples, but the present invention is not limited to the following description.

### <Synthesis of NMN from D-ribose>

### [Example 1]

Recombinants with enhanced expression of ScRbk (derived from Saccharomyces cerevisiae), BsPrs (derived from Bacillus subtilis), HdNampt (derived from Haemophilus ducreyi), and DrPpk (derived from Deinococcus radiodurans), which were constructed according to the methods described in Examples of PCT International Publication No. WO2019/065876, were used. In addition, for PPase, a reagent product (derived from yeast, manufactured by Sigma-Aldrich Co. LLC, product number: 10108987001) was used.

### (1) Preparation of cell-free extract

The culture solution of each recombinant prepared according to the method described in PCT International Publication No. WO2019/065876 was centrifuged (5000 × g, 10 minutes), and the supernatant was discarded. A 50 mM potassium phosphate buffer (pH 7.8) was added to the precipitated bacterial cells, and the turbidity at a wavelength of 630 nm was adjusted to 125. The adjusted bacterial cell suspension in buffer was centrifuged again (5000 × g, 10 minutes), the supernatant was discarded, and then a 50 mM potassium phosphate buffer (pH 7.8) in an amount equal to that of the supernatant was added to obtain a washed bacterial cell suspension in buffer having a turbidity of 125 at a wavelength of 630 nm. The obtained bacterial cell suspension in buffer was subjected to disruption (100 MPa × 2 times) with a tabletop high-pressure homogenizer Panda PLUS 2000 manufactured by GEA Niro Soavi S.p.A. The bacterial cell suspension in buffer was cooled on ice and then subjected to a high-pressure homogenizer, and the obtained disrupted solution was also immediately cooled on ice. The disrupted solution was centrifuged (5000 × g, 10 minutes), and the obtained supernatant was used as a cell-free extract. The protein concentration of the cell-free extract was measured using bovine serum albumin (BSA, available from Bio-Rad Laboratories, Inc.) as a standard protein and a Bio-Rad protein assay (available from Bio-Rad Laboratories, Inc.).

### (2) NMN synthesis reaction

An NMN synthesis reaction was carried out using the cell-free extract obtained in (1). The volume of the reaction solution was set to 300 mL, and the raw material compounds were heated to 37°C with the formulation shown in Table 1. Thereafter, the reaction mixture was stirred at a rotation speed of 300 rpm, and 15 mmol of magnesium hydroxide (Mg(OH)₂) as a pH-maintaining agent was added thereto to adjust the pH to 7.5 to 7.8, after which the cell-free extract was added to start the reaction. At each of 2 hours, 4 hours, and 6 hours after the start of the reaction, 1.5 mmol of polyphosphate (as a 25% aqueous solution) was added thereto. The polyphosphate used was sodium metaphosphate manufactured by FUJIFILM Wako Pure Chemical Corporation, assuming that the molecular weight thereof was 1388 g/mol. During the reaction, a total of 49 mmol of Mg(OH)₂ powder was added thereto in four separate portions as a pH-maintaining agent to maintain the pH of the reaction solution at 6.5 to 8.0.

### (3) Analysis of NMN

The analysis of the NMN synthesis reaction sample was carried out by HPLC under the following conditions.
Column: InertSustain AQ-C18 (5 µm, 150 × 4.6 mm I.D., manufactured by GL Sciences Inc.)
Mobile phase: 50 mM potassium phosphate buffer pH 7.5
Flow rate: 1.5 mL/min
Detection: UV 261 nm
Column temperature: 40°C

### (4) Experimental results

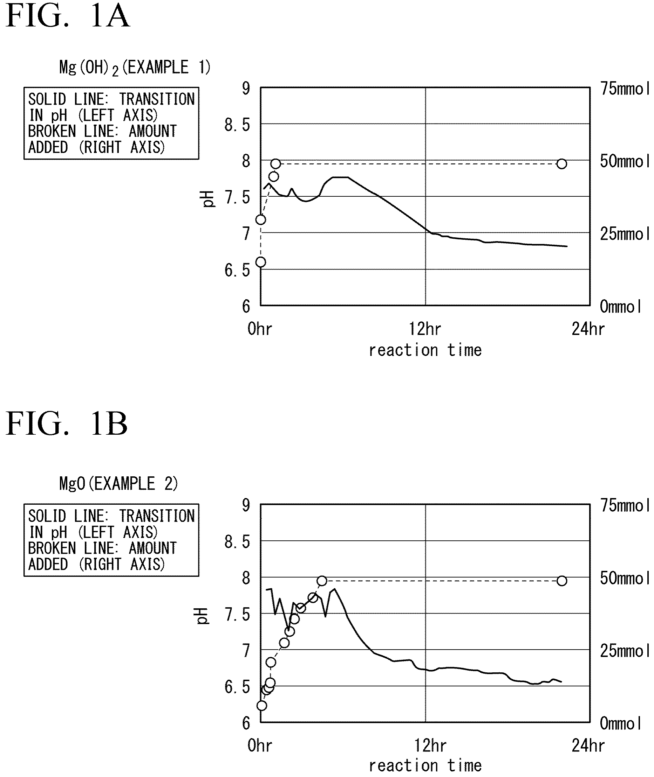

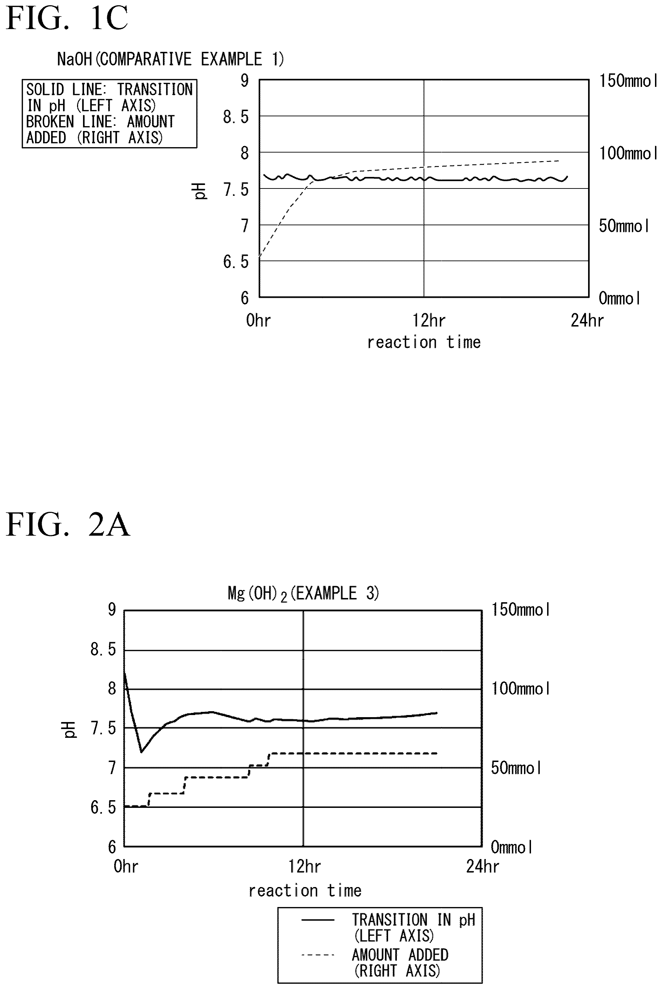

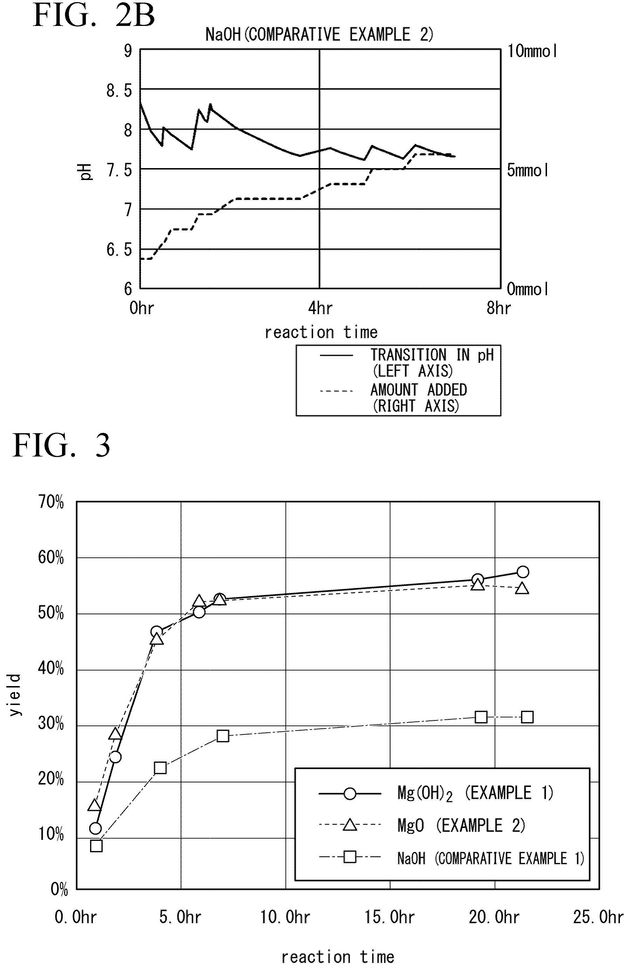

FIG. 1A shows an amount of a pH-maintaining agent (Mg(OH)₂) added over time (broken line) and a transition in pH (solid line). FIG. 2 shows a transition in NMN synthesis yield in a case where the pH is maintained by a pH-maintaining agent (Mg(OH)₂). For the NMN synthesis yield, the amount of NMN substance in the reaction solution was calculated from the NMN concentration quantified under the above-mentioned NMN analysis conditions using NMN manufactured by Oriental Yeast Co., Ltd. as a standard reagent, and the value obtained by dividing the calculated value by the amount of Ribose and NAM substances charged (22.5 mmol) was used.

### [Example 2]

NMN was synthesized in the same manner as in Example 1, except that magnesium oxide (MgO) was used instead of magnesium hydroxide as the pH-maintaining agent and was added in 11 separate portions.

FIG. 1B shows an amount of a pH-maintaining agent (MgO) added over time (broken line) and a transition in pH (solid line). FIG. 3 shows a transition in NMN synthesis yield in a case where the pH was maintained by a pH-maintaining agent (MgO).

### [Comparative Example 1]

NMN was synthesized in the same manner as in Example 1, except that a 25% sodium hydroxide aqueous solution was used as the pH-maintaining agent, the pH was adjusted to 7.8 by adding the pH-maintaining agent, and then the reaction was started by adding a cell-free extract, and a total of 94 mmol of a 25% sodium hydroxide aqueous solution was continuously added during the reaction to maintain the pH at 7.5 to 8.0.

FIG. 1C shows an amount of a pH-maintaining agent (NaOH) added over time (broken line) and a transition in pH (solid line). FIG. 3 shows a transition in NMN synthesis yield in a case where the pH was maintained by a pH-maintaining agent (NaOH).

**[Table 1]**

| | Reaction solution composition (final concentration) | Example 1 | Example 2 | Comparative Example 1 |
|---|---|---|---|---|
| Raw material compounds | Ribose [mM] | 75 | 75 | 75 |
| | Nicotinamide [mM] | 75 | 75 | 75 |
| | Magnesium chloride [mM] | 50 | 50 | 50 |
| | Polyphosphate [mM] | 15 | 15 | 15 |
| | ATP·2Na trihydrate [mM] | 1.5 | 1.5 | 1.5 |
| Cell-free extract | ScRbk [g/L] | 0.06 | 0.06 | 0.06 |
| | BsPrs [g/L] | 0.065 | 0.065 | 0.065 |
| | HdNampt [g/L] | 1.4 | 1.4 | 1.4 |
| | DrPpk [g/L] | 2.8 | 2.8 | 2.8 |
| | PPase [g/L] | 0.005 | 0.005 | 0.005 |
| pH-maintaining agent | Mg(OH)₂ [mmol] | 49 | - | - |
| | MgO [mmol] | - | 49 | - |
| | NaOH [mmol] | - | - | 94 |
| Additional additive raw material | Polyphosphate [mmol] | 4.5 | 4.5 | 4.5 |

As shown in FIG. 1A to FIG. 1C, in Comparative Example 1 in which NaOH was used as the pH-maintaining agent, the pH was maintained with higher accuracy than in Examples 1 and 2 in which Mg(OH)₂ or MgO was used as the pH-maintaining agent. On the other hand, as shown in FIG. 3, the NMN synthesis yield 21 hours after the start of the reaction was 58% in Example 1, 55% in Example 2, and 32% in Comparative Example 1, and NMN could be produced with a higher yield in a case where the pH of the reaction solution was adjusted with Mg(OH)₂ or MgO than in a case where the pH of the reaction solution was adjusted with NaOH.

In Examples 1 and 2, three types of enzymes using ATP, Rbk, Prs, and Nampt, coexisted. The reactions catalyzed by these enzymes proceed in a cascade manner, and a product of a certain enzymatic reaction acts as a substrate for a subsequent enzymatic reaction. From this, it was shown that the effect of the present invention is highly likely to act on any of the reactions of Rbk, Prs, and Nampt.

As shown in Table 1, in any of Examples 1 and 2 and Comparative Example 1, 33 equivalents of Mg ions with respect to ATP were added to the raw material compounds, and a sufficient amount of divalent metal ions coexisted in the reaction solution. From this, the decrease in the NMN synthesis yield in Comparative Example 1 was not due to a shortage of Mg ions in the reaction solution.

### <Synthesis of carnosine>

### [Example 3]

A recombinant with enhanced expression of a mutant enzyme (N108Q/Il 12V/H378R) of Bacillus subtilis-derived Lal (BsLal), which was constructed according to the method described in Japanese Unexamined Patent Application, First Publication No. 2020-22433, was used. In the construction of the mutant enzyme YwfE (mutant type: N108Q/I112V/H378R), based on the sequence information described in Japanese Unexamined Patent Application, First Publication No. 2020-22433, the present inventors requested GenScript Biotech Corporation (https://www.genscript.jp/?src=google&utm_source=google&utm_medium=cpc&utm_ca mpaign=GenScript&gad_source=1&gclid=EAIaIQobChMI9fyp4PXwhAM V-gh7Bx0axQxGEAAYASAAEgI0v_D_BwE) to synthesize a gene and clone the synthesized gene into a pET21a(+) vector to obtain a target plasmid, and then transformed the obtained target plasmid into Escherichia coli BL21 (DE3) competent cells to construct a recombinant. For a recombinant with enhanced expression of DrPpk (derived from Deinococcus radiodurans), the recombinant was constructed using a method according to Example 1, and then a cell-free extract was prepared.

### (1) Preparation of cell-free extract

The culture solution of the above-mentioned recombinant prepared according to the method described in Examples of PCT International Publication No. WO2019/065876 was centrifuged (5000 × g, 10 minutes), and the supernatant was discarded. A 50 mM potassium phosphate buffer (pH 7.8) was added to the precipitated bacterial cells, and the turbidity at a wavelength of 630 nm was adjusted to 50. The adjusted bacterial cell suspension in buffer was centrifuged again (5000 × g, 10 minutes), the supernatant was discarded, and then a 50 mM potassium phosphate buffer (pH 7.8) in an amount equal to that of the supernatant was added to obtain a washed bacterial cell suspension in buffer having a turbidity of 50 at a wavelength of 630 nm. The obtained bacterial cell suspension in buffer was subjected to disruption using an ultrasonic homogenizer (VP-300N) manufactured by Taitec Corporation (20% output, treatment time: 7 min). The bacterial cell suspension in buffer was cooled on ice and then subjected to ultrasonic disruption, and the obtained disrupted solution was also immediately cooled on ice. The disrupted solution was centrifuged (5000 × g, 10 minutes), and the obtained supernatant was used as a cell-free extract. The protein concentration of the cell-free extract was measured using bovine serum albumin (BSA, available from Bio-Rad Laboratories, Inc.) as a standard protein and a Bio-Rad protein assay (available from Bio-Rad Laboratories, Inc.).

### (2) Carnosine synthesis reaction

A carnosine synthesis reaction was carried out using the cell-free extract obtained in (1). The volume of the reaction solution was set to 30 mL, and the raw material compounds were heated to 30°C with the formulation shown in Table 2. Thereafter, the reaction mixture was stirred at a rotation speed of 50 rpm, and 3.4 mmol of magnesium hydroxide (Mg(OH)₂) as a pH-maintaining agent was added thereto to adjust the pH to 8.0 to 8.2, after which the cell-free extract was added to start the reaction. During the reaction, a total of 7.9 mmol of Mg(OH)₂ powder was sequentially added thereto as a pH-maintaining agent to maintain the pH of the reaction solution at 7.6 to 8.2.

### (3) Analysis of carnosine

The analysis of the carnosine synthesis reaction sample was carried out by HPLC under the following conditions.
Column: Shodex Asahipak NH2P-50 4E (4.6 mm × 250 mm, manufactured by Resonac Holdings Corporation)
Mobile phase: 50 mM NaH₂PO₄:CH₃CN = 40:60
Flow rate: 1.0 mL/min
Detection: UV 210 nm
Column temperature: 40°C

### (4) Experimental results

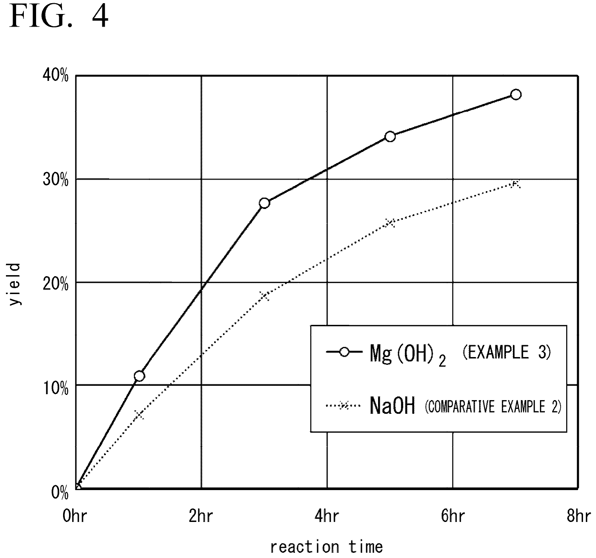

FIG. 2A shows an amount of a pH-maintaining agent (Mg(OH)₂) added over time (broken line) and a transition in pH (solid line). FIG. 4 shows a transition in carnosine synthesis yield in a case where the pH was maintained by a pH-maintaining agent (Mg(OH)₂). For the carnosine synthesis yield, the amount of L-carnosine substance in the reaction solution was calculated from the L-carnosine concentration quantified under the above-mentioned L-carnosine analysis conditions using L-carnosine manufactured by FUJIFILM Wako Pure Chemical Corporation as a standard reagent, and the value obtained by dividing the calculated value by the amount of β-alanine and L-histidine substances charged (2.25 mmol) was used.

### [Comparative Example 1]

Carnosine was synthesized in the same manner as in Example 1, except that a 25% sodium hydroxide aqueous solution was used as the pH-maintaining agent, the pH was adjusted to 7.8 by adding the pH-maintaining agent, and then the reaction was started by adding a cell-free extract, and a total of 5.6 mmol of sodium hydroxide was continuously added during the reaction to maintain the pH at 7.6 to 8.3.

FIG. 2B shows an amount of a pH-maintaining agent (NaOH) added over time (broken line) and a transition in pH (solid line). FIG. 4 shows a transition in carnosine synthesis yield in a case where the pH was maintained by a pH-maintaining agent (NaOH).

**[Table 2]**

| | Reaction solution composition (final concentration) | Example 3 | Comparative Example 2 |
|---|---|---|---|
| Raw material compounds | β-alanine [mM] | 75 | 75 |
| | L-histidine [mM] | 75 | 75 |
| | Magnesium chloride [mM] | 50 | 50 |
| | Polyphosphate [mM] | 20 | 20 |
| | ATP·2Na trihydrate [mM] | 2.0 | 2.0 |
| Cell-free extract | YwfE (N108Q/I112V/H378R) [g/L] | 2.5 | 2.5 |
| | DrPpk [g/L] | 2.0 | 2.0 |
| pH-maintaining agent | Mg(OH)₂ [mmol] | 7.9 | - |
| | NaOH [mmol] | - | 5.6 |

As shown in FIG. 4, the L-carnosine synthesis yield 7 hours after the start of the reaction was 38% in Example 3 and 30% in Comparative Example 1, and L-carnosine could be produced with a higher yield in a case where the pH of the reaction solution was adjusted with Mg(OH)₂ than in a case where the pH of the reaction solution was adjusted with NaOH.

As shown in Table 2, in any of Example 3 and Comparative Example 2, 25 equivalents of Mg ions with respect to ATP were added to the raw material compounds, and a sufficient amount of divalent metal ions coexisted in the reaction solution. From this, the decrease in the L-carnosine synthesis yield in Comparative Example 2 was not due to a shortage of Mg ions in the reaction solution.

### <Synthesis of NMN from D-ribose>

### [Example 4]

A recombinant with enhanced expression of XtNampt derived from Xanthomonas translucens was constructed according to the method described in [Example 1], and then a cell-free extract was prepared in the same manner as in [Example 1]. The sequence information of XtNampt was acquired from a sequence database (UniProt, No. A0A0K2ZNB0).

### (1) NMN synthesis reaction

An NMN synthesis reaction was carried out using the above-mentioned XtNampt. The volume of the reaction solution was set to 300 mL, and the raw material compounds were heated to 37°C with the formulation shown in Table 3. Thereafter, the reaction mixture was stirred at a rotation speed of 300 rpm, and 11 mmol of magnesium hydroxide (Mg(OH)₂) as a pH-maintaining agent was added thereto to adjust the pH to 7.5 to 7.8, after which the cell-free extract was added to start the reaction. At each of 2 hours, 4 hours, and 6 hours after the start of the reaction, 1.5 mmol of polyphosphate (as a 25% aqueous solution) was added thereto. The polyphosphate used was sodium metaphosphate manufactured by FUJIFILM Wako Pure Chemical Corporation, assuming that the molecular weight thereof was 1388 g/mol. During the reaction, a total of 49 mmol of Mg(OH)₂ powder was added thereto in four separate portions as a pH-maintaining agent to maintain the pH of the reaction solution at 6.5 to 8.0.

### (2) Experimental results

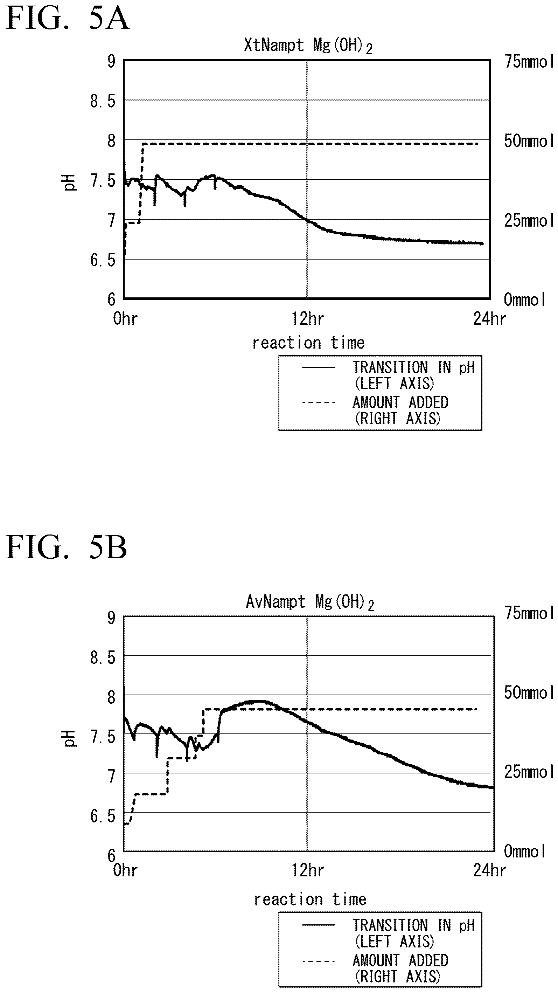

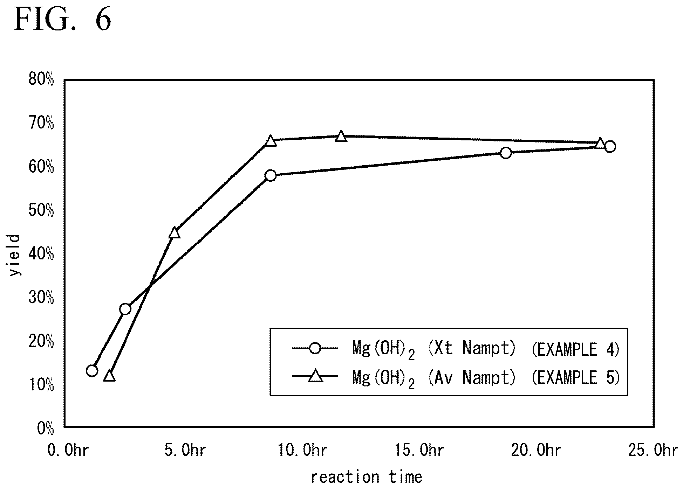

FIG. 5A shows an amount of a pH-maintaining agent (Mg(OH)₂) added over time (broken line) and a transition in pH (solid line). The analysis conditions were the same as those in the section of "(3) Analysis of NMN" of Example 1, the amount of NMN substance in the reaction solution was calculated from the quantified NMN concentration, and the value obtained by dividing the calculated value by the amount of Ribose and NAM substances charged (22.5 mmol) was used as the yield. The results of the reaction are shown in FIG. 6.

### [Example 5]

A recombinant with enhanced expression of AvNampt derived from Aquimonas voraii was constructed according to the method described in [Example 1], and then a cell-free extract was prepared in the same manner as in [Example 1]. The sequence information of AvNampt was acquired from a sequence database (UniProt, No. A0A1G6Z2F3).

### (1) NMN synthesis reaction

An NMN synthesis reaction was carried out using the above-mentioned AvNampt. The volume of the reaction solution was set to 300 mL, and the raw material compounds were heated to 37°C with the formulation shown in Table 3. Thereafter, the reaction mixture was stirred at a rotation speed of 300 rpm, and 9 mmol of magnesium hydroxide (Mg(OH)₂) as a pH-maintaining agent was added thereto to adjust the pH to 7.5 to 7.8, after which the cell-free extract was added to start the reaction. At each of 2 hours, 4 hours, and 6 hours after the start of the reaction, 1.5 mmol of polyphosphate (as a 25% aqueous solution) was added thereto. The polyphosphate used was sodium metaphosphate manufactured by FUJIFILM Wako Pure Chemical Corporation, assuming that the molecular weight thereof was 1388 g/mol. During the reaction, a total of 45 mmol of Mg(OH)₂ powder was added thereto in five separate portions as a pH-maintaining agent to maintain the pH of the reaction solution at 6.5 to 8.0.

### (2) Experimental results

FIG. 5B shows an amount of a pH-maintaining agent (Mg(OH)₂) added over time (broken line) and a transition in pH (solid line). The analysis conditions were the same as those in the section of "(3) Analysis of NMN" of Example 1, the amount of NMN substance in the reaction solution was calculated from the quantified NMN concentration, and the value obtained by dividing the calculated value by the amount of Ribose and NAM substances charged (22.5 mmol) was used as the yield. The results of the reaction are shown in FIG. 6.

In Examples 4 and 5, NMN could be produced with a synthesis yield equal to or higher than that of Examples 1 and 2.

**[Table 3]**

| | Reaction solution composition (final concentration) | Example 4 | Example 5 |
|---|---|---|---|
| Raw material | Ribose [mM] | 75 | 75 |
| compounds | Nicotinamide [mM] | 75 | 75 |
| | Magnesium chloride [mM] | 50 | 50 |
| | Polyphosphate [mM] | 15 | 15 |
| | ATP·2Na trihydrate [mM] | 1.5 | 1.5 |
| Cell-free extract | ScRbk [g/L] | 0.06 | 0.06 |
| | BsPrs [g/L] | 0.065 | 0.065 |
| | XtNampt [g/L] | 1.4 | - |
| | AvNampt [g/L] | - | 1.4 |
| | DrPpk [g/L] | 2.8 | 2.8 |
| | PPase [g/L] | 0.005 | 0.005 |
| pH-maintaining agent | Mg(OH)₂ [mmol] | 49 | 45 |
| | MgO [mmol] | - | - |
| | NaOH [mmol] | - | - |
| Additional additive raw material | Polyphosphate [mmol] | 4.5 | 4.5 |

## Claims

1. A method for producing a substance by an enzymatic reaction using adenosine 5'-triphosphate, the method comprising:
carrying out the enzymatic reaction and an ATP regeneration reaction in which adenosine 5'-diphosphate or adenosine 5'-monophosphate is reacted with a phosphate donor to generate adenosine 5'-triphosphate, in one reaction solution in the presence of a basic compound containing a divalent metal.

2. A method for producing a substance, comprising:
producing β-nicotinamide mononucleotide using D-ribose, nicotinamide, and adenosine 5'-triphosphate in the presence of ribokinase, phosphoribosyl pyrophosphate synthetase, nicotinamide phosphoribosyltransferase, polyphosphate kinase, and a basic compound containing a divalent metal.

3. A method for producing a substance by an enzymatic reaction using adenosine 5'-triphosphate, the method comprising:
carrying out the enzymatic reaction and an ATP regeneration reaction in which adenosine 5'-diphosphate or adenosine 5'-monophosphate is reacted with a phosphate donor to generate adenosine 5'-triphosphate, in one reaction solution,
wherein the pH of the reaction solution is adjusted with a basic compound containing a divalent metal.

4. The method for producing a substance according to any one of Claims 1 to 3, wherein the basic compound is at least one selected from basic compounds containing magnesium, calcium, strontium, barium, manganese, iron, cobalt, nickel, or zinc as the divalent metal.

5. The method for producing a substance according to any one of Claims 1 to 3, wherein the basic compound containing a divalent metal is at least one selected from magnesium hydroxide and magnesium oxide.

6. The method for producing a substance according to Claim 1 or 3, wherein the ATP regeneration reaction is an ATP regeneration reaction by polyphosphate kinase.

7. The method for producing a substance according to Claim 1 or 3, wherein the enzymatic reaction is an enzymatic reaction using at least one enzyme selected from enzymes belonging to EC number EC 2.4.-, EC 2.5.-, EC 2.7.-, EC 6.2.-, or EC 6.3.-.

8. The method for producing a substance according to Claim 6, wherein the enzymatic reaction is an enzymatic reaction using at least one enzyme selected from L-amino-acid alpha-ligase, ribokinase, phosphoribosyl pyrophosphate synthetase, and nicotinamide phosphoribosyltransferase.

9. The method for producing a substance according to Claim 6, wherein the enzymatic reaction is an enzymatic reaction in which ribokinase, phosphoribosyl pyrophosphate synthetase, and nicotinamide phosphoribosyltransferase are combined.

10. The method for producing a substance according to Claim 6, wherein the enzymatic reaction is an enzymatic reaction in which xylose isomerase, xylulokinase, ribulose-5-phosphate-3-epimerase, ribose-5-phosphate isomerase, phosphoribosyl pyrophosphate synthetase, and nicotinamide phosphoribosyltransferase are combined.

11. The method for producing a substance according to Claim 1 or 3, wherein the total concentration of adenosine 5'-triphosphate, adenosine 5'-diphosphate, and adenosine 5'-monophosphate in the reaction solution is 0.1 mM to 3 mM.

12. The method for producing a substance according to Claim 1 or 3, wherein the pH of the reaction solution is maintained at 6.0 to 9.0 using the basic compound containing a divalent metal.

13. The method for producing a substance according to Claim 1 or 3, wherein the substance produced by the enzymatic reaction is at least one selected from a nucleotide, an amino acid, a peptide, and a phosphate compound.

14. The method for producing a substance according to Claim 1 or 3, wherein the substance produced by the enzymatic reaction is β-nicotinamide mononucleotide, inosine monophosphate, carnosine, aspartic acid, glutathione, oxidized glutathione, γ-glutamylcysteine, phosphoribosyl pyrophosphate, ribose-5-phosphate, glycerol triphosphate, acetyl CoA, or S-adenosylmethionine.
